Europäisches Patentamt

(19) European Patent Office

Office européen des brevets

(11) Publication number: **0 068 379**
**B1**

(12) **EUROPEAN PATENT SPECIFICATION**

(45) Date of publication of patent specification: **13.02.85**

(21) Application number: **82105415.2**

(22) Date of filing: **21.06.82**

(51) Int. Cl.⁴: **G 01 N 31/22**, G 01 N 33/96

(54) **Analytical control device and method for making it.**

(30) Priority: **29.06.81 US 278385**

(43) Date of publication of application:
**05.01.83 Bulletin 83/01**

(45) Publication of the grant of the patent:
**13.02.85 Bulletin 85/07**

(84) Designated Contracting States:
**DE FR GB**

(56) References cited:
**DE-A-2 835 296**
**US-A-3 963 442**

(73) Proprietor: **MILES LABORATORIES, INC.**
**1127 Myrtle Street**
**Elkhart Indiana 46514 (US)**

(72) Inventor: **Rapkin, Myron Coleman**
**156 Briggs Beaumont**
**Texas 77707 (US)**

(74) Representative: **Jesse, Ralf-Rüdiger, Dr. et al**
**Bayer AG Konzernverwaltung RP**
**Patentabteilung**
**D-5090 Leverkusen 1 Bayerwerk (DE)**

Courier Press, Leamington Spa, England.

## Description

### Field of the invention

The present invention relates to a device for preparing an analytical control solution of an analyte, said device comprising a base support member having affixed thereto an analyte composition, a process for making such a device and to a process for preparing one analytical control solution of an analyte.

When a procedure is devised for determining the presence of a sample constituent—be the devised procedure gravimetric, volumetric, spectrophotometric or whatever mode—its efficacy in producing reliable results must somehow be assessed. Otherwise, the data developed is meaningless. Hence, devising an analytical procedure extends far beyond building a machine, formulating reagents or developing a technique. It also must of necessity include evaluating experimental error. There must be a way of predicting the dependability of the data produced by the procedure.

The easiest, most direct way to study parameters such as reproducibility, sensitivity, accuracy and need for calibration is to subject the procedure to a test sample wherein the analyte presence and/or concentration is known beforehand, i.e., a control solution. The data furnished by the procedure can then be compared with known data and any discrepancies properly noted.

The present invention, therefore, concerns itself with assessing procedures for determining the presence and/or concentration of an analyte in a solution. Moreover, it relates to a device for preparing an analytical control solution of an analyte for use in evaluating various analytical procedures.

### Description of the prior art

Exemplary of the state-of-the-art prior to the present invention is a product currently marketed by the Ames Division of Miles Laboratories, Inc. known as CHEK-STIX®. It consists of various pads of filter paper mounted on a strip of plastic, each pad being impregnated with one or more substances which, when dissolved in water, simulate pathological urine constituents. Thus, when a CHEK-STIX device is immersed in a premeasured volume of water for a predetermined time, a control solution results which simulates urine containing such metabolites as glucose, bilirubin, urobilinogen, ketone, occult blood, nitrite and protein. This control solution can be used to monitor the test device known as N-MULTISTIX®, a dip-and-read analytical device marketed by the Ames Division of Miles Laboratories, Inc.

Yet another prior art approach in preparing control solutions is embodied by the product TEK-CHEK®, also marketed by the Ames Division of Miles Laboratories, Inc. It comprises lyophilized urine containing natural and artificial additives to simulate both normal and pathological urine. In use, TEK-CHEK is added to a premeasured quantity of water to form a control solution.

The present invention differs from the closest prior art known, CHEK-STIX, in that it eliminates the necessity of using paper pads, thereby markedly shortening the time required to form the control solution. In addition, the present invention is easier to manufacture, because it is not necessary to impregnate and dry filter paper pads.

### Summary of the invention

The present invention comprises a new device for preparing a control solution as disclosed in claim 6, as well as a novel process for making the device. The process comprises preparing a coating solution by combining a water-soluble polymer, an analyte an etchant substance and a solvent therefore. The coating solution is then applied to a portion of a base support member. The support member comprises a substance capable of being etched by the etchant substance to form a composite. Finally, the composite is dried.

The device comprises a base support member having affixed to a portion of its surface an analyte composition. The analyte composition comprises a water-soluble polymer and an analyte, and is affixed to the base support member so as to be partially, but not completely, embedded in the surface of the support member. A process for preparing an analytical control solution is disclosed in claim 9.

### Detailed description of the invention

The presently claimed device is useful for formulating control solutions containing a broad diversity of analytes. Exemplary of such analytes are ascorbic acid, bilirubin, blood (both red and white cells, as well as other blood components), sugars (such as gluclose), ketones, salicylate, nitrite ion, protein and urobilinogen. As can be seen from the many chemically unrelated analytes exemplified, *supra*, the scope of analytes useful in the present invention is indeed broad, and the determination of whether or not a particular analyte is compatible with the invention is fully within the capability of the routineer, given the instant teachings. Moreover, it is equally apparent that pseudoanalytes are intended as within the scope of the term "analyte" as used herein, i.e. materials which imitate another substance in an analytical procedure. Thus for example, where a procedure is designed to respond to the presence of acetone, a highly volatile analyte, a useful control solution might comprise a higher molecular weight ketone normally solid at room temperature. Such a pseudoanalyte could be used to advantage in the present invention because of enhanced shelf life: acetone would evaporate, whereas the pseudoanalyte would not.

The device of the present invention is produced by forming a coating solution and applying it to a base support member such as an oblong plastic strip. The coating solution comprises a solution or slurry, in a suitable solvent (i.e, liquid vehicle), of a water-soluble polymer, the analyte and an etchant substance.

As a suitable solvent, a wide range of liquids are useful. In many cases, the etchant substance itself can be used, as will be apparent from the examples. Selecting the proper solvent— be it water, benzene, ethanol, acetone, xylene or other solvent—is a matter of routine experimentation, once the present teachings have been digested.

The water-soluble polymer used herein can range widely, provided it satisfy the requirements of solubility and compatibility with the other components of the coating solution and the ultimate device. Preferred water-soluble polymers are ethyl hydroxyethylcellulose, guar gum, carboxymethyl cellulose polyacrylamide, poly (diallyldimethylammonium chloride) and poly (ethyleneimine). Especially suitable is carboxymethyl cellulose.

The etchant substance used in preparing the invention must be capable of partially etching the base support member without, however, dissolving it during the application of the coating solution to the base support member. For example, where the base support member is composed of polystyrene, suitable etchant substances are toluene, cyclohexane and benzene. In a preferred embodiment of the invention the etchant substance is an aromatic liquid. especially preferred as the etchant substance is toluene.

The selection of material for use as the base support member is limited solely by its propensity to be etched by the etchant substance, and by its insolubility in water. Generally, however, it is preferred to use a rigid or semirigid homopolymer or copolymer material such as polystyrene, polyethylene and other polyolefins, nylon. In a preferred embodiment the base support material is a biaxially oriented polystyrene strip, such as that obtainable from Plastic Suppliers Inc.

Because of the coaction between the etchant substance and the base support member, the coating solution becomes at least partially, but not completely, embedded in the base support member. This highly desirable phenomenon enables excellent adhesion of the analyte to the support member. It is this unexpectedly strong adhesion that is presently believed to provide the ease of forming control solutions which the present invention affords, and its enhanced ease of manufacture. Moreover, because the analyte is so tightly bound to the base support, the device has excellent shelf life.

Although the preferred process for preparing the control device of the present invention is clearly set forth in the examples below, the basic procedure comprises forming the coating solution, applying it to the base support member, and drying the resulting composite. The coating is generally applied to one end of an oblong shaped support member, the other end serving as a handle. Preferably, a relatively large (as compared to the ultimate intended size of a single device) piece of polystyrene sheet is coated along its longest dimension by drawing the coating solution out with a doctor blade, porous roller, brush or other suitable applicator. When the solution has dried, the composite is slit parallel to its short dimension to yield devices wherein the analyte is at one end, the other serving as a handle.

Examples
The following examples are provided in order to set forth the presently envisioned best mode of making and using the present invention. While they illustrate presently preferred embodiments, they are in no way to be interpreted as limiting the scope of the invention.

Example I
Glucose control device
An experiment was conducted whereby a device was prepared for use in making an analytical control solution for glucose analysis.

A coating solution was prepared by combining the following ingredients in the order as listed:

| | |
|---|---|
| Glucose (60 g/100 ml water) | 5.0 milliliters (ml) |
| Carboxymethyl cellulose (Hercules, Inc. No. 81480) | 0.1 gram (g) |
| Toluene | 0.5 ml |

This mixture was poured onto a sheet of polystyrene measuring 4"×24" (obtained from American Can Co., Inc.), which served as the base support member, and was spread with an applicator known as a Mayer Rod. Mayer Rods are steel rods wrapped tightly with varying gauges of stainless steel wire. The thickness of an applied coating is dependent on the diameter of the wrapping wire. Mayer rods are frequently used in the coating industry and may be obtained from R & D Specialties in Webster, New York.

The coated polystyrene was dried at room temperature. The thus-formed control device is used to prepare a glucose-containing control solution by immersing the coated end in 12 ml water for 2 minutes and removing the device. The resultant solution yields a positive test for glucose when tested with N-MULTISTIX reagent strips.

Example II
Bilirubin control device
An experiment was conducted whereby a device was prepared for use in making an analytical control solution for bilirubin analysis.

A coating solution was prepared by combining the following ingredients in the order as listed:

| | |
|---|---|
| Bilirubin (obtained from Pfansthiel, Inc.) | 0.10 g |
| Sodium hydroxide (0.01 N in water) | 5.0 ml |
| Carboxymethyl Cellulose (Hercules, Inc. Lot No. 81480) | 1.0 g |
| Toluene | 0.05 ml |

This mixture was applied to a polystyrene sheet as in Example I.

The thus-formed control device is used to prepare a bilirubin-containing control solution by immersing it into 12 ml of water for 2 minutes and removing the device. The resultant solution gives a positive test for bilirubin when tested with N-MULTISTIX reagent strips.

## Claims

1. A process for making a device for preparing an analytical control solution of an analyte, characterised by the steps of
preparing a coating solution by combining a water-soluble polymer, the analyte, an etchant substance, and a solvent therefor,
applying the coating solution to a base support member comprising a substance capable of being etched by the etchant substance to form a composite, and
drying the composite.

2. The process of claim 1 wherein the water soluble polymer is ethyl hydroxyethylcellulose, guar gum, or preferably carboxymethyl cellulose.

3. The process of claims 1 or 2 wherein the etchant is an aromatic solvent.

4. The process of claims 1 or 2 wherein the etchant is toluene and the support member is polystyrene.

5. The process of claims 1 or 2 wherein the analyte is glucose, bilirubin, nitrite, ketone, hemoglobin, salicylate or albumin.

6. A device for preparing an analytical control solution of an analyte, said device comprising a base support member having affixed thereto an analyte composition characterised in that the analyte composition comprises a water soluble polymer and the analyte, said composition being at least partially, but not completely, embedded by an action of an etchant substance in the surface of said support member.

7. The device of claim 6 wherein the water soluble polymer is ethyl hydroxyethylcellulose, guar gum or preferably carboxymethyl cellulose.

8 The device of claims 6 or 7 wherein the support member is polystyrene.

9. A process for preparing an analytical control solution of an analyte, said process comprising immersing the device of claims 6 or 7 into water for a time sufficient to permit said analyte to diffuse from said device into said water.

10. The process of claim 9 in which said device is immersed in a premeasured volume of said water for a predetermined period of time and then removed from said water.

## Revendications

1. Un procédé pour fabriquer un dispositif pour préparer une solution de contrôle analytique d'un analyte, caractérisé par les étapes suivantes:
on prépare une solution de revêtement en combinant un polymère soluble dans l'eau, l'analyte, une substance décapante et un solvant de ceux-di,
on applique la solution de revêtement sur un élément de support de base consistant en une substance susceptible d'être attaquée par la substance décapante pour former un matériau composite, et
on sèche le matériau composite.

2. Le procédé de la revendication 1, dans lequel le polymère soluble dans l'eau est l'ethylhydroxyéthylcellulose, la gomme guar ou de préférence la carboxyméthylcellulose.

3. Le procédé des revendications 1 ou 2, dans lequel la substance décapante est un solvant aromatique.

4. Le procédé des revendications 1 ou 2, dans lequel la substance décapante est le toluène et l'élément de support est le polystyrène.

5. Le procédé des revendications 1 ou 2, dans lequel l'analyte est le glucose, la bilirubine, un nitrite, une cétone, l'hémoglobine, un salicylate ou l'albumine.

6. Un dispositif pour préparer une solution de contrôle analytique d'un analyte, ledit dispositif consistant en un élément de support de base sur lequel est fixée une composition d'analyte, caractérisé en ce que la composition d'analyte consiste en un polymère soluble dans l'eau et l'analyte, ladite composition étant au moins partiellement, mais pas complètement, noyée dans la surface dudit élément de support par l'action d'une substance décapante.

7. Le dispositif de la revendication 6, dans lequel le polymère soluble dans l'eau est l'éthylhydroxyéthylcellulose, la gomme guar ou de préférence la carboxyméthylcellulose.

8. Le procédé des revendications 6 ou 7, dans lequel l'élément de support est du polystyrène.

9. Un procédé pour préparer une solution de contrôle analytique d'un analyte, ledit procédé consistant à plonger dans l'eau le dispositif des revendications 6 ou 7 pendant une durée suffisante pour permettre audit analyte de diffuser dudit dispositif dans ladite eau.

10. Le procédé de la revendication 9, dans lequel ledit dispositif est plongé dans un volume préalablement mesuré de ladite eau pendant une durée prédéterminée et ensuite retiré de ladite eau.

## Patentansprüche

1. Verfahren zur Herstellung einer Vorrichtung für die Zubereitung einer analytischen Kontrollösung eine zu analysierenden Substanz, gekennzeichnet durch die Stufen:

Zubereitung einer Beschichtungslösung durch Kombinieren eines wasserlöslichen Polymers, der zu analysierenden Substanz, eines Ätzmittels und eines Lösungsmittels dafür,

Aufbringen der Beschichtungslösung auf einen Grundträgerteil aus einer Substanz, die in der Lage ist, unter Ausbildung eines Verbundstoffes durch das Ätzmittel geätzt zu werden und

Trocknen der Verbundstoffes.

2. Verfahren gemäss Anspruch 1, worin das wasserlösliche Polymer Ethylhydroxycellulose, Guarharz oder vorzugsweise Carboxymethylcellulose ist.

3. Verfahren gemäss Ansprüchen 1 oder 2, worin das Ätzmittel ein aromatisches Lösungsmittel ist.

4. Verfahren gemäss Ansprüchen 1 oder 2, worin das Ätzmittel Toluol und der Trägerteil Polystyrol ist.

5. Verfahren gemäss Ansprüchen 1 oder 2, worin die zu analysierende Substanz Glucose, Bilirubin, Nitrit, Keton, Hämoglobin, Salicylat oder Albumin ist.

6. Vorrichtung zur Herstellung einer analytischen Kontrollösung für eine zu analysierende Substanz, wobei die Vorrichtung eine Grundträgerteil mit einer daran befestigten Zusammensetzung der zu analysierenden Substanz enthält, dadurch gekennzeichnet, dass die Zusammensetzung der zu analysierenden Substanz ein wasserlösliches Polymer und die zu analysierende Substanz enthält und die Zusammensetzung wenigstens zum Teil, aber nicht vollständig durch die Einwirkung eines Ätzmittels in die Oberfläche des Trägerteils eingebettet ist.

7. Vorrichtung gemäss Anspruch 6, worin das wasserlösliche Polymer Ethylhydroxyethylcellulose, Guarharz oder vorzugsweise Carboxymethylcellulose ist.

8. Vorrichtung gemäss Ansprüchen 6 oder 7, worin das Trägerteil Polystyrol ist.

9. Verfahren zur Herstellung einer analytischen Kontrollösung einer zu analysierenden Substanz, wobei das Verfahren das Eintauchen der Vorrichtung gemäss Ansprüchen 6 und 7 in Wasser während einer ausreichenden Zeit, um die zu analysierende Substanz aus der Vorrichtung in Wasser diffundieren zu lassen, umfasst.

10. Verfahren gemäss Anspruch 9, bei dem die Vorrichtung in einem vorher abgemessenen Volumen des Wassers während eines vorbestimmten Zeitraums eingetaucht und dann aus dem Wasser entfernt wird.